# EUROPEAN PATENT SPECIFICATION

(11) **EP 1 251 737 B1**
(45) Date of publication and mention of the grant of the patent: **06.04.2005**
(21) Application number: 01905076.4
(22) Date of filing: 25.01.2001
(51) Int. Cl.: A01N 33/04, A01N 37/44, A01N 41/02, A01N 47/28, A01N 47/44, A01N 47/48, A61L 2/18, A61K 31/785

(54) **COMPOSITIONS TREATED TO INACTIVATE INFECTIOUS PROTEINS**
ZUR INAKTIVIERUNG VON INFEKTIÖSEN PROTEINEN BEHANDELTE ZUSAMMENSETZUNGEN
COMPOSITIONS TRAITEES POUR INACTIVER LES PROTEINES INFECTIEUSES

(30) Priority: 31.01.2000 US 494814; 26.10.2000 US 699284
(43) Date of publication of application: 30.10.2002
(73) Proprietor: The Regents of the University of California, Oakland, CA 94607-5200 (US)
(72) Inventor: PRUSINER, Stanley, B., San Francisco, CA 94123 (US); SUPATTAPONE, Surachai, New Hampshire 03755 (US); SCOTT, Michael, R., San Francisco, CA 94114 (US)
(74) Representative: Campbell, Patrick John Henry
(86) International application number: PCT/US2001/002555
(87) International publication number: WO 2001/054736

(56) References cited:
- WO-A-00/72851
- DE-A- 3 229 097
- US-A- 5 633 349
- US-A- 5 780 288
- DATABASE BIOSIS [Online] BIOSCIENCES INFORMATION SERVICE, PHILADELPHIA, PA, US; 7 December 1999 (1999-12-07) SUPATTAPONE SURACHAI ET AL: "Elimination of prions by branched polyamines and implications for therapeutics." Database accession no. PREV200000056439 XP002191399 & PROCEEDINGS OF THE NATIONAL ACADEMY OF SCIENCES OF THE UNITED STATES, vol. 96, no. 25, 7 December 1999 (1999-12-07), pages 14529-14534, Dec. 7, 1999 ISSN: 0027-8424

## Description

### FIELD OF THE INVENTION

The present invention relates generally to compositions used to inactivate infectious proteins such as infectious prions.

### BACKGROUND TO THE INVENTION

Antiseptic compositions have been known for over 100 years. In addition to various compositions there are a range of different methods which are known to be effective in killing bacteria and inactivating viruses. Such methods include the use of high temperature alone or in combination with radiation over sufficient periods of time to kill bacteria or disrupt viruses and thereby inactivate them.

Examples of fast acting topical antiseptic compositions are disclosed within U.S. Patent 6,110,908 issued August 29, 2000. Another antibacterial composition is disclosed within U.S. Patent 6,025,312 issued February 15, 1000. Examples of other antiseptic compositions are taught within U.S. Patents 5,336,432 issued August 9, 1994; 5,308,611 issued May 3, 1994; 6,106,773 issued August 22, 2000 and 6,096,216 issued August 1, 2000.

Conventional antiseptic compositions and antiseptic methodologies are generally insufficient for inactivating infectious proteins such as prions. Although prions can be inactivated by relatively high temperatures over very long periods of time the temperature ranges and time periods generally used to kill bacteria and inactive the viruses are insufficient to inactive prions. One approach to solving this problem is to attempt to remove prions from solutions and a chromographic removal process is disclosed within U.S. Patent 5,808,011. Further, others have attempted to provide compositions and methodologies which are intended to inactive prions as is taught within U.S. Patent 5,633,349 issued May 27, 1997. However, such processes generally take relatively long periods of time e.g. more than 12 hours and generally do not provide a solution which could be readily and economically utilized in order to inactivate prions on a wide range of surfaces such as medical devices. In addition, WO 00/72851 discloses a method of sterilizing objects which involves contacting the object with polycationic dendrimer under conditions which result in rendering a conformationally altered protein, such as a prion, non-infectious. Supattapone *et al* (P.N.A.S. 96 pages 14529-14534, 1999) describes the removal of prion proteins from scrapie-infected neuroblastoma cells in culture.

The present invention offers an antiseptic composition and method for inactivating prions as described further below.

### BRIEF DESCRIPTION OF THE DRAWINGS

Figure 1 is a schematic drawing of a dendrimer molecule showing the defined "generations" ofhomodisperse structure created using a repetitive divergent growth technique. The specific diagram is ofPAMAM, generation 2.0 (ethylene diamine core).
Figure 2 includes gel panels 2A (2 hours) and 2B (5 minutes). Within Figure 2A column 4 (+) shows that SDS at a pH of about 3.3. completely eliminates PrP^{c} whereas 1% SDS at a pH of 7.0 is not effective. Figure 2B within column 4 shows that 1% SDS at a pH of 3.3. is effective in inactivating prions after only five minutes of exposure.
Figure 3 includes a gel panel wherein columns show results carried out at different temperatures indicating that the optimal temperature for using acetic SDS to eliminate prions is greater than 20°C.
Figure 4 shows four separate gels with each of the gels run at nine different specific pH levels showing that SDS does denature PrP^{Sc} at low pH and high pH but not at a relatively neutral pH and further showing that increasing the percent concentration of SDS improves the ability of the formulation to denature PrP^{Sc}.
Figure 5 provides gel panels which show that acetic buffers other than acetic acid and sodium acetate can be used in combination with SDS in order to denature PrP^{Sc}.

### SUMMARY OF THE INVENTION

An antiseptic composition is used to completely inactivate infectious prions. The composition is comprised of a first agent which maintains the pH of the composition in the range of from 2.5 to 4.5; and a second agent comprising a detergent characterized by its ability to destroy infectivity of infectious prion in the low pH environment created by the first component (e.g. in two hours or less). The first agent may be any well known acid present in an aqueous solution at sufficient molarity so as to reduce the pH of the antiseptic composition to a value in the range of from 2.5 to 4.5 and maintain the pH at that low level when the antiseptic composition is applied to an object or mixed with a material to be treated. The composition will vary due to the large number of different acids and detergent inactivating agents that can be used. As temperature is increased and pH is lowered the inactivation occurs more rapidly. Compositions of the invention preferably inactivate prions in about 2 hours or less at a pH of 2.5 to 4.0 at temperatures of about 15°C to 40°C. However, low temperatures (e.g. 0°C or higher) and high temperatures (e.g. 100°C or less) can be used as can longer time periods. The inactivation can occur more quickly (e.g. in one minute or less) and at higher temperatures (e.g. greater than 40°C).

The antiseptic composition is preferably combined with an additional component which results in the formation of treated food, pharmaceutical material or an object selected from a pharmaceutical gel capsule, a gel coated tablet, a blood extender or blood replacement solution, a surgical implant, a bandage, a suture, a dental implement, a dental sponge, a surgical sponge, a candy containing a gelatin such as a caramel candy, a marshmallow, or a mint such as an Altoid® mint, doughnut glaze, fruit juice, wine, beer, sour cream, yogurt, cottage cheese, ice cream, margarine and chewing gum. In essence, the antiseptic composition can be combined with or coated onto any material which may include infectious prions and particularly with any material comprising animal products, most particularly bovine products such as bovine gelatin.

A method is disclosed whereby any type of object can be sterilized by combining normal sterilization procedures with the use of the antiseptic composition disclosed herein which is capable of rendering a conformationally altered protein such as a prion non-infectious. The method is particularly useful in sterilizing medical devices such as surgical instruments and catheters which have been used and brought into contact with blood or brain tissue. Objects sterilized via the method are also part of the invention and include capsules which are made from gelatin extracted from cattle which cattle may be infected with prions, i.e. have undiagnosed BSE known as "mad cow disease".

The antiseptic compositions can be combined with conventional antibacterial and antiviral agents in aqueous or alcohol solutions to produce disinfecting agents or surgical scrubs. The essence of the invention disclosed here is that a wide range of different types of detergents will render prions non-infectious in a relatively short period of time (e.g. 2 hours or less) when maintained at a pH of 2.5 to 4.5 at moderate temperatures, e.g. 15° to 30° centigrade. In some cases the commercial value of the invention is decreased if the composition does not accomplish its intended purpose in a short time period (e.g. less than 10 minutes at about room temperature 20°C ± 5°C).

An overall aspect of the invention is a method of treating a material to inactivate infectious prions, comprising the steps of:
contacting the material with a composition, comprising:
   a solvent;
   an acid component characterised by and present in a molarity so as to maintain the pH of the composition in a range of from 2.5 to 4.5; and
   a detergent; and
allowing the composition to remain in contact with the material which completely inactivates infectious prions.

An advantage of the invention is that proteins such as prions can be rendered non-infectious without the need for extreme conditions such as exposure to heat over long periods of time, e.g. without the need for an exposure of 1 to 10 hours at 100 to 200°C.

A feature of the invention is that compositions can be useful while containing only very low concentrations of the prion inactivating component such as SDS, eg. 1% to 0.001%.

Another aspect of the invention is that capsules made with bovine gelatin can be certified prion free.

Another aspect of the invention is that drugs produced from cell cultures treated with a detergent such as SDS can be certified prion free.

Still another aspect of the invention is that medical devices being reused after exposure to blood or brain tissue can be certified prion free.

Still another aspect of the invention is that hospitals, operating rooms and devices and equipment within them can be certified prion free by contacting them with detergents at standard temperatures and pressures.

An advantage of the invention is that conformationally altered protein such as prions can be rendered non-infectious with a method which need only consist of applying an active component such as SDS preferably held at a pH of from 2.5 to 4.5.

Another aspect of the invention is soaps, surgical scrubs, detergents and the like are formulated to contain the acid component and the detergent prion inactivating component.

Other aspects of the invention are methods of sterilizing manufacturing equipment such as chromatographic columns and using the sterilized equipment to manufacture products such as pharmaceuticals.

Another aspect is the use of compositions to sterilize dental instruments, equipment and offices.

An advantage of the invention is that compositions comprised of the detergent inactivating component and acid component can be used to inactivate prions which might be present on surgical instruments, knives and/or other tools or equipment used by butchers, particularly those used in the butchering of cows or other animals which might be infected with prions.

A feature of the invention is that compositions of the invention can be effective in inactivating prions when the detergent inactivating component (e.g. SDS) is present in very low concentrations, e.g. 1% to 0.001% or less.

### DETAILED DESCRIPTION OF PREFERRED EMBODIMENTS

Before the present methods, objects and compositions are described, it is to be understood that this invention is not limited to the particular steps, devices or components described and, as such, may of course vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### DEFINITIONS

The term "acid" is used to describe any compound or group of compounds which has one or more characteristics of (a) sour taste; (b) turns litmus dye red; (c) reacts with certain metals to form a salt; (d) reacts with certain bases or alkalines to form a salt. An acid comprises hydrogen and in water undergoes ionization so that H₃O⁺ ions are formed - also written as H⁺ and referred to as hydronium ions or simply hydrogen ions. Weak acids such as acetic acid or carbonic acid may be used as may strong acids such as hydrochloric acid, nitric acid and sulfuric acid (HCl, H₂SO₄, H₂NO₃). In compositions of the invention the acid is preferably present in a concentration so as to obtain a pH of from 2.5 to 4.5. The acid component of the antiseptic composition must be present in a concentration (molarity) to keep the composition in the desired pH range. The concentration (molarity) or the acid used will vary somewhat with the particular acid used, the solvent used (water or alcohol) and other factors such as temperature and pressure. The acid component is preferably present in sufficient molarity and is of such type that when the antiseptic composition of the invention is put into use (e.g. mixed with a sample to be disinfected) the composition remains within the preferred pH range. Thus, stronger and/or more concentrated forms of acids are preferred when the composition is to be used on or in a situation where the composition will be significantly diluted and/or contact a high pH (i.e. very basic component).

The terms "active component," "active agent," "inactivating agent" and the like are used interchangeably herein to describe a compound or group of compounds which when combined in the "acid" component of the invention in the antiseptic composition will render a conformationally altered protein non-infectious. The active component is within an environment of a pH of from 2.5 to 4.5 and preferably in a low concentration, e.g. less than 5% by volume of the composition, and will inactivate prions or other conformationally altered proteins in two hours or less at about room temperature 15°C to 30°C. Preferred detergents which act as an active component for prions include SDS.

The active compounds of the present invention are the following detergents:
a) sodium dodecyl sulfate (SDS) (also known as lauryl sulfate, sodium salt - other salts are also useful including lithium and potassium salts).
b) sodium cholate
c) sodium deoxycholate
d) octylglucoside
e) dodecyldimethylamine oxide
f) 3-[(3-cholamidopropyl)dimethylammonio]-1-propanesulfonate (CHAPS)
g) dodecyltriethylammonium bromide (DTAB)
h) cetyltrimethylammonium bromide (CTAB)
i) polyoxyethylene-p-isooctylphenyl ether (e.g. Triton X-20, Triton X-100, Triton X-114).

An aspect of the invention is providing the compound group above with a material which may contain infectious prions under conditions which inactivate the infectivity of the prions, which conditions preferably comprise a pH of from 2.5 to 4.5 and a temperature of 15° C or higher, wherein the inactivation takes place in a period of time of 2 hours or less, preferably 1 hour or less and more preferably 10 minutes or less.

Further information of compounds and conditions effecting protein conformation can be found in Voet et. al., Biochemistry, pgs 180-280 (1990); Scopes, RK., Protein Purification: Principles and Practice, pgs. 57-71 (1987); and Deutscher, MP., Guide to Protein Purification, pgs. 240-241 (1990).

The term "detergent" is used to mean any substance that reduces the surface tension of water. Examples of detergents are provided above as possible active components. The detergent may be a surface active agent which concentrates at oil-water interfaces, exerts emulsifying action and thereby aids in removing soils e.g. common sodium soaps of fatty acids. A detergent may be anionic, cationic, or monionic depending on their mode of chemical action. Detergents include linear alkyl sulfonates (LAS) often aided by "builders." A LAS is preferably an alkyl benzene sulfonate ABS which is readily decomposed by microorganisms (biodegradable). The LAS is generally a straight chain alkyl comprising 10 to 30 carbon atoms. The detergent may be in a liquid or a solid form.

The terms "prion", "prion protein", "PrP^{Sc} protein" and the like are used interchangeably herein to refer to the infectious PrP^{Sc} form of a PrP protein, and is a contraction of the words "protein" and "infection." Particles are comprised largely, if not exclusively, of PrP^{Sc} molecules encoded by a PrP gene. Prions are distinct from bacteria, viruses and viroids. Known prions infect animals to cause scrapie, a transmissible, degenerative disease of the nervous system of sheep and goats, as well as bovine spongiform encephalopathy (BSE), or "mad cow disease", and feline spongiform encephalopathy of cats. Four prion diseases known to affect humans are (1) kuru, (2) Creutzfeldt-Jakob Disease (CJD), (3) Gerstmann-Sträussler-Scheinker Disease (GSS), and (4) fatal insomnia (FI). As used herein "prion" includes all forms of prions causing all or any of these diseases or others in any animals used - and in particular in humans and domesticated farm animals.

The term "conformationally altered protein" is used here to describe any protein which has a three dimensional conformation associated with a disease. The conformationally altered protein may cause the disease, be a factor in a symptom of the disease or appear as a result of other factors. The conformationally altered protein appears in another conformation which has the same amino acid sequence. In general, the conformationally altered protein formed is "constricted" in conformation as compared to the other "relaxed" conformation which is not associated with disease. Those skilled in the art reading this disclosure will recognize the applicability of the antiseptic composition of the invention to other conformationally altered proteins even though the invention is described in general regards to prions. The following is a non-limiting list of diseases with associated proteins which assemble two or more different conformations wherein at least one conformation is an example of a conformationally altered protein.

| Disease | Insoluble Proteins |
|---|---|
| Alzheimer's Disease | APP, Aβ peptide, α1-antichymotrypsin, tau, non-Aβ component, presenillin 1, presenillin 2 apoE |
| Prion diseases, Creutzfeldt Jakob disease, scrapie and bovine spongiform encephalopathy | PrP^{Sc} |
| ALS | SOD and neurofilament |
| Pick's disease | Pick body |
| Parkinson's disease | α-synuclein in Lewy bodies |
| Frontotemporal dementia | tau in fibrils |
| Diabetes Type II | Amylin |
| Multiple myeloma― plasma cell dyscrasias | IgGL-chain |
| Familial amyloidotic polyneuropathy | Transthyretin |
| Medullary carcinoma of thyroid | Procalcitonin |
| Chronic renal failure | β₂―microglobulin |
| Congestive heart failure | Atrial natriuretic factor |
| Senile cardiac and systemic amyloidosis | Transthyretin |
| Chronic inflammation | Serum amyloid A |
| Atherosclerosis | ApoA1 |
| Familial amyloidosis | Gelsolin |
| Huntington's disease | Huntingtin |

The terms "sterilizing", "making sterile" and the like are used here to mean rendering something non-infectious or rendering something incapable of causing a disease. Specifically, it refers to rendering a protein non-infectious or incapable of causing a disease or the symptoms of a disease. Still more specifically, it refers to rendering a conformationally altered protein (e.g. PrP^{Sc} known as prions) incapable of causing a disease or the symptoms of a disease.

By "effective dose" or "amount effective" is meant an amount of a compound sufficient to provide the desired sterilizing result, *e.g.*, eliminate the infectivity of any prions present. This will vary depending on factors such as (1) the active agent used, (2) the pH of the antiseptic composition, (3) the type of object or material being sterilized, and (4) the amount or concentration of infectious proteins which might be present. The concentration is sufficient if the resulting composition is effective in decreasing (preferably eliminating) the infectivity of conformationally altered proteins such that the treated material over tune would not result in infection. Because (1) some materials will have higher concentrations of altered protein than others (2) some materials are contacted more frequently than others and (3) individual proteins have different degrees of infectivity the effective dose or concentration range needed to sterilize can vary considerably. It is also pointed out that the dose needed to treat an amount of material may vary somewhat based on the pH the treatment is carried out at and the amount of time the compound is maintained in contact with the material at the desired low pH (2.5 to 4.5) level and the surrounding temperature and pressure.

The term "LD₅₀" as used herein is the dose of an active substance that will result in 50 percent lethality in all treated experimental animals. Although this usually refers to invasive administration, such as oral, parenteral, and the like, it may also apply to toxicity using less invasive methods of administration, such as topical applications of the active substance.

The term "amine-terminated" includes primary, secondary and tertiary amines.

The terms "PrP protein", "PrP" and like are used interchangeably herein and shall mean both the infectious particle form PrP^{Sc} known to cause diseases (spongiform encephalopathies) in humans and animals and the noninfectious form PrP^{C} which, under appropriate conditions is converted to the infectious PrP^{Sc} form.

The term "PrP gene" is used herein to describe genetic material which expresses proteins including known polymorphisms and pathogenic mutations. The term "PrP gene" refers generally to any gene of any species which encodes any form of a prion protein. Some commonly known PrP sequences are described in Gabriel et al., *Proc. Natl. Acad. Sci. USA* 89:9097-9101 (1992) and U.S. Patent No. 5,565,186. The PrP gene can be from any animal, including the "host" and "test" animals described herein and any and all polymorphisms and mutations thereof, it being recognized that the terms include other such PrP genes that are yet to be discovered. The protein expressed by such a gene can assume either a PrP^{C} (non-disease) or PrP^{Sc} (disease) form.

The terms "standardized prion preparation", "prion preparation", "preparation" and the like are used interchangeably herein to describe a composition (e.g., brain homogenate) obtained from the brain tissue of mammals which exhibits signs of prion disease: the mammal may (1) include a transgene as described herein; (2) have and ablated endogenous prion protein gene; (3) have a high number of prion protein gene from a genetically diverse species; and/or (4) be a hybrid with an ablated endogenous prion protein gene and a prion protein gene from a genetically diverse species. Different combinations of 1-4 are possible, *e*.*g*., 1 and 2. The mammals from which standardized prion preparations are obtained exhibit clinical signs of CNS dysfunction as a result of inoculation with prions and/or due to developing the disease of their genetically modified make up, *e*.*g*., high copy number of prion protein genes. Standardized prion preparations and methods of making such are described and disclosed in U.S. Patent 5,908,969 issued June 1, 1999 and U.S. Patent 6,020,537 issued February 1, 2000.

The term "Alzheimer's disease" (abbreviated herein as "AD") as used herein refers to a condition associated with formation of neuritic plaques comprising amyloid β protein, primarily in the hippocampus and cerebral cortex, as well as impairment in both learning and memory. "AD" as used herein is meant to encompass both AD as well as AD-type pathologies.

The term "AD-type pathology" as used herein refers to a combination of CNS alterations including, but not limited to, formation of neuritic plaques containing amyloid β protein in the hippocampus and cerebral cortex. Such AD-type pathologies can include, but are not necessarily limited to, disorders associated with aberrant expression and/or deposition of APP, overexpression of APP, expression of aberrant APP gene products, and other phenomena associated with AD, Exemplary AD-type pathologies include, but are not necessarily limited to, AD-type pathologies associated with Down's syndrome that is associated with overexpression of APP.

The term "phenomenon associated with Alzheimer's disease" as used herein refers to a structural, molecular, or functional event associated with AD, particularly such an event that is readily assessable in an animal model. Such events include, but are not limited to, amyloid deposition, neuropathological developments, learning and memory deficits, and other AD-associated characteristics.

The term "cerebral amyloid angiopathy" (abbreviated herein as CAA) as used herein refers to a condition associated with formation of amyloid deposition within cerebral vessels which can be complicated by cerebral parenchymal hemorrhage. CAA is also associated with increased risk of stroke as well as development of cerebellar and subarachnoid hemorrhages (Vinters (1987) *Stroke* 18:311-324; Haan et al. (1994) *Dementia* 5:210-213; Itoh et al. (1993) *J Neurol. Sci*. 116:135-414). CAA can also be associated with dementia prior to onset of hemorrhages. The vascular amyloid deposits associated with CAA can exist in the absence of AD, but are more frequently associated with AD.

The term "phenomenon associated with cerebral amyloid angiopathy" as used herein refers to a molecular, structural, or functional event associated with CAA, particularly such an event that is readily assessable in an animal model. Such events include, but are not limited to, amyloid deposition, cerebral parenchymal hemorrhage, and other CAA-associated characteristics.

The term "β-amyloid deposit" as used herein refers to a deposit in the brain composed of Aβ as well as other substances.
Abbreviations used herein include:
CNS for central nervous system;
BSE for bovine spongiform encephalopathy;
CJD for Creutzfeldt-Jakob Disease;
FFI for fatal familial insomnia;
GSS for Gerstmann-Sträussler-Scheinker Disease;
AD for Alzheimer's disease;
CAA for cerebral amyloid angiopathy;
Hu for human;
HuPrP for human prion protein;
Mo for mouse;
MoPrP for mouse prion protein;
SHa for a Syrian hamster;
SHaPrP for a Syrian hamster prion protein;
PAMAM for polyamidoamide dendrimers
PEI for polyethyleneimine
PK for proteinase K
PPI for polypropyleneimine
PrP^{Sc} for the scrapie isoform of the prion protein;
PrP^{C} for the cellular contained common, normal isoform of the prion protein;
PrP 27-30 or PrP^{Sc} 27-30 for the treatment or protease resistant form of PrP^{Sc};
MoPrP^{Sc} for the scrapie isoform of the mouse prion protein;
N2a for an established neuroblastoma cell line used in the present studies;
ScN2a for a chronically scrapie-infected neuroblastoma cell line;
ALS for amyotrophic lateral sclerosis;
HD for Huntington's disease;
FTD for frontotemporal dementia;
SDS for sodium dodecyl sulfate;
SOD for superoxide dismutase

### GENERAL ASPECTS OF THE INVENTION

The invention uses a range of antiseptic compositions and methods of rendering conformationally altered proteins non-infectious. The composition is comprised of an acid component and a detergent although a single compound could serve the function of both components. The composition comprises a solvent carrier component which is generally alcohol or aqueous based. The acid component is characterized by maintaining the pH of the composition in the range of from 2.5 to 4.5 when in use. The detergent is characterized by rendering infectious proteins completely non-infectious. Preferably the active component in the low pH environment of the composition renders infectious proteins non-infectious in two hours or less at a temperature of 40° centigrade or less.

Suitable acid components include a non-toxic weak acid such as acetic acid having dissolved therein the detergent. The antiseptic compositions are coated on, mixed with, injected into or otherwise brought into contact with a material to be sterilized. The composition is maintained in the desired pH range at normal temperature (e.g., 15°C to 30°C) for a sufficient period of time (e.g. less than 2 hours) to cause conformationally altered protein present on or in the material to be destroyed (e.g. hydrolyzed) or rendered non-infective. Preferred compositions of the invention are useful in cleaning and sterilizing and may be comprised of an active agent such as SDS and an acid component providing a pH less than 3.5.

### DENDRIMER COMPOUNDS WHICH CLEAR PRIONS

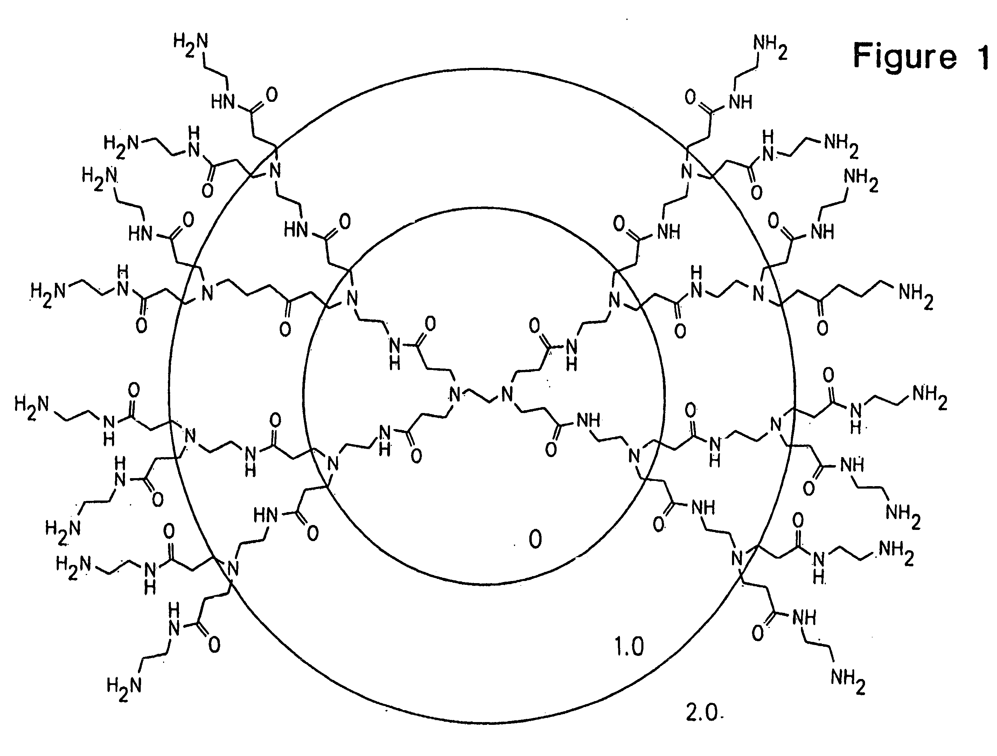

Dendrimers are branched compounds also known as "starburst" or "star" polymers due to a characteristic star-like structure (see Figure 1). Dendrimers which may be added to the compositions of the invention are polymers with structures built from ABₙ monomers, with n≥2, and preferably n=2 or 3. Such dendrimers are highly branched and have three distinct structural features: 1) a core, 2) multiple peripheral end-groups, and 3) branching units that link the two. Dendrimers may be cationic (full generation dendrimers) or anionic (half generation dendrimers). For a review on the general synthesis, physical properties, and applications of dendrimers, see, *e*.*g*., Tomalia et. al, Angew. *Chem. Int. Ed*. *Engl*., 29:138-175, (1990); Y. Kim and C. Zimmerman, *Curr Opin Chem Biol,* 2:733-7421 (1997).

Sterilizing compositions may comprise a cationic dendrimer preferably dissolved in a low pH solvent such as acetic acid. Examples of suitable dendrimers are disclosed in U.S. Pat. Nos. 4,507,466, 4,558,120, 4,568,737, 4,587,329, 4,631,337, 4,694,064, 4,713,975, 4,737,550, 4,871,779, and 4,857,599 to D. A. Tomalia, et al. Dendrimers typically have tertiary amines which have a pKa of 5.7. The dendrimers can optionally be chemically or heat treated to remove some of the tertiary amines. Other suitable cations include polypropylene imine, polyethyleneimine (PEI), which has tertiary amines with a pKa of 5.9, and poly(4'-aza-4'-methylheptamethylene D-glucaramide), which has tertiary amines with a pKa of 6.0. The cationic dendrimer is preferably dissolved in the low pH solvent such as vinegar in a concentration of 0.0001% or more, preferably 0.01% or more and more preferably about 1%.

Preferably, the dendrimers are polyamidoamines (hereinafter "PAMAM"). PAMAM dendrimers are particularly biocompatible, since polyamidoamine groups resemble peptide bonds of proteins.

Dendrimers are prepared in tiers called generations (see generations 0, 1 and 2 in Figure 1) and therefore have specific molecular weights. The full generation PAMAM dendrimers have amine terminal groups, and are cationic, whereas the half generation dendrimers are carboxyl terminated. Full generation PAMAM dendrimers are thus preferred for use in the present invention. PAMAM dendrimers may be prepared having different molecular weights and have specific values as described in Table A below for generations 0 through 10.

**TABLE A**

| LIST OF PAMAM DENDRIMERS AND THEIR MOLECULAR WEIGHTS (Ethylene Diamine core, amine terminated), | | |
|---|---|---|
| GENERATION | TERMINAL GROUPS | MOL. WT. g/mole |
| 0 | 4 | 517 |
| 1 | 8 | 1430 |
| 2 | 216 | 3256 |
| 3 | 32 | 6909 |
| 4 | 64 | 14,215 |
| 5 | 128 | 28,795 |
| 6 | 256 | 58,048 |
| 7 | 512 | 116,493 |
| 8 | 1024 | 233,383 |
| 9 | 2048 | 467,162 |
| 10 | 4096 | 934,720 |

As shown in Table A, the number of terminal amine groups for PAMAM dendrimers generations 0 through 10 range from 4 to 4,096, with molecular weights of from 517 to 934,720. PAMAM dendrimers are available commercially from Aldrich or Dendritech. Polyethyleneimine or polypropylene dendrimers or quaternized forms of amine-terminated dendrimers may be prepared as described by Tomalia et. al, Angew, *Chem*. *Int*. *Ed. Engl*., 29:138-175 (1990).

### STERILIZING COMPOSITIONS

Examples provided here show that various active compounds such as SDS at a pH of 4.0 or less affect the extent and distribution of PrP^{Sc} protein deposits in scrapie-infected cells. The presence of these active compounds in a low pH environment and at relatively low, non-cytotoxic levels results in a significant reduction in detectable PrP^{Sc} in cells and brain homogenates. Thus, the present invention encompasses use of compositions for completely inactivating the infectivity of a protein. A composition used in the invention is comprised of any detergent capable of destroying conformationally altered proteins when in a low pH environment (e.g. SDS) in solution, suspension or mixture.

### STERILIZING FORMULATIONS.

Sterilizing compositions preferably contain the detergent in a concentration from 0.0001 to 10% of the formulation. The following methods and excipients are merely exemplary and are in no way limiting.

In addition to including the detergent compound in the formulation it is important to maintain that compound in a low pH environment. Any number of known acids or mixtures of acids could be used with the invention. Non-limiting examples of commercially available products which could be supplemented into the compositions are described below. In these formulations the percentage amount of each ingredient can vary. In general a solvent ingredient (e.g. water or alcohol) is present in amounts of 40% to 100%. The other ingredients are present in an amount in a range of 1% to 60% and more generally 5% to 20%. The amount added is an amount needed to obtain the desired effect.

| **FORMULATION 1** | |
|---|---|
| **Component** | **wt %** |
| water | 10 - 99 |
| acid | 1 - 20 |
| detergent | 0.01 - 10 |

| **FORMULATION 2** | |
|---|---|
| **Component** | **wt %** |
| water | 10 - 98 |
| acid | 1 - 20 |
| detergent | 1 - 20 |
| polycationic dendrimer | 0.01 - 5 |

| **FORMULATION 3** | |
|---|---|
| **Component** | **wt %** |
| water | 10 - 98 |
| acetic acid | 1 - 20 |
| linear alkyl sulfonate | 1 - 20 |
| polycationic dendrimer | 0.01 - 5 |

| **FORMULATION 4** | |
|---|---|
| **Component** | **wt %** |
| water | 10 - 99 |
| acetic acid | 1 - 20 |
| SDS | 0.01 - 10 |

| **FORMULATION 5** | |
|---|---|
| **Component** | **wt %** |
| water | 1 - 98 |
| alcohol | 0 - 98 |
| acid | 1 - 20 |
| detergent | 1 - 20 |
| polycationic dendrimer | 0.1 - 5 |

| **FORMULATION 6** | |
|---|---|
| **Component** | **wt %** |
| water | 1 - 99 |
| acid | 1 - 20 |
| antibacterial | 0.1 - 5 |
| detergent | 1 - 20 |
| polycationic dendrimer | 0.1 - 5 |

| **FORMULATION 7** | |
|---|---|
| **Component** | **wt %** |
| water | 3 - 98.889 |
| antimicrobial active agent | 0.001-5 |
| anionic surfactant | 1 - 80 |
| proton (H⁺) donating agent | 0.1 - 12 |
| polycationic dendrimer | 0.01 - 5 |

| **FORMULATION 8** | |
|---|---|
| **Component** | **wt %** |
| water | 1 - 98 |
| alcohol | 0 - 98 |
| acid | 1 - 20 |
| SDS | 1 - 20 |

| **FORMULATION 9** | |
|---|---|
| **Component** | **wt %** |
| water | 1 - 99 |
| acid | 1 - 20 |
| antibacterial agent | 0.1 - 5 |
| detergent | 1 - 20 |

| **FORMULATION 10** | |
|---|---|
| **Component** | **wt %** |
| water | 3 - 98.889 |
| antimicrobial active agent | 0.001 - 5 |
| anionic surfactant | 1 - 80 |
| proton (H⁺) donating agent | 0.1 - 12 |
| SDS | 0.01 - 5 |

| **FORMULATION 11** | |
|---|---|
| **Component** | **wt %** |
| SDS | 0.5 |
| Ethanol | 74.0 |
| Benzalkonium chloride | 0.2 |
| CAE | 0.02 |
| Glycerine | 1.0 |
| Chain silicone | 0.5 |
| Triglyceride | 0.5 |
| Lactic acid | 10.0 |
| Purified water | 13.28 |

| **FORMULATION 12** | |
|---|---|
| **Component** | **wt %** |
| Sodium deoxycholate | 0.01 |
| Ethanol | 75.0 |
| Benzalkonium chloride | 0.2 |
| CAE | 0.02 |
| Glycerine | 2.0 |
| Chain silicone | 0.99 |
| Cyclic silicone | 2.0 |
| Triglyceride | 3.0 |
| Lactic acid | 9 |
| Purified water | 7.78 |

| **FORMULATION 13** | |
|---|---|
| **Component** | **wt %** |
| SDS | 1 |
| Ethanol | 75.0 |
| Chlorhexedine gluconate | 0.2 |
| Benzalkonium chloride | 0.2 |
| CAE | 0.02 |
| Glycerine | 0.8 |
| Chain silicone | 0.2 |
| Cyclic silicone | 0.2 |
| Triglyceride | .38 |
| Acetic acid | 10 |
| Purified water | 12 |

| **FORMULATION 14** | |
|---|---|
| **Component** | **wt %** |
| SDS | 0.001 |
| Ethanol | 75.99 |
| Chlorhexedine gluconate | 0.2 |
| CAE | 0.02 |
| Glycerine | 1.0 |
| Chain silicone | 0.2 |
| Triglyceride | 0.3 |
| Lactic acid | 14 |
| Purified water | 8.28 |

| **FORMULATION 15** | |
|---|---|
| **Component** | **wt %** |
| Sodium Acetate pH 4.0 ± | 10% |
| SDS | 4% |
| Water | 86% |

| **FORMULATION 16** | |
|---|---|
| **Component** | **wt %** |
| SDS | 4% |
| Peracetic acid | 0.1 -10% |
| Water | 86 - 95.9% |

| **FORMULATION 17** | |
|---|---|
| **Component** | **wt %** |
| SDS | 1 - 20% |
| Acid pH 4.0 ± 1 | 1 -20% |
| water | 60 - 98% |

By using the disclosure provided here and other information such as taught in U.S. Patents 5,767,054; 6,007,831; 5,830,488; 5,968,539; 5,416,075; 5,296,158; and patents and publications cited therein those skilled in the art can produce countless other formulations of the invention. Further, the formulations are preferably adjusted to have a pH of less than 4.0 and such formulations can be used as described in such publications and can be packaged in any suitable container or dispenser device, e.g. taught in 5,992,698. The pH can be lowered with any acid, e.g. HCl, H₂SO₄, H₂NO₃ peracetic acid, etc. can be used as the acid component in the formulae provided above.

Example 3 shows that compounds such as SDS are effective in denaturing PrP^{Sc} not only at a low pH (e.g. 5 or less) but are effective at a high pH (e.g. 9 or more) but are generally not effective at a pH of about 7.0 ± 1. The pH of the formulation can be adjusted to obtain desired results in each particular use. For example, a very high or very low pH may be best for inactivating PrP^{Sc}, but extreme pH's may be undesirable in some situations due to their corrosive effects. Thus, a preferred pH is one which inactivates PrP^{Sc} and has the least possible adverse effects for the intended use.

Formulations used with a cell culture have the advantage that they are non-toxic. For example, parenteral administration of a solution of the formulations of the invention is preferably nontoxic at a dosage of 0. 1 mg/mouse, which is an LD₅₀ of less than one at 40 mg/Kg. Various nutrient formulations and/or injectable formulations of the type known to those skilled in the art can be used to prepare formulations for treating cell cultures.

Those skilled in the art will understand that in some situations it may be desirable to further reduce the pH environment to obtain the desired results. This can be accomplished by adding any desired acid. If desired, the pH can be raised to a normal level after treatment is complete, i.e. after a sufficient amount of any conformationally altered protein present are destroyed.

Compounds effective in sterilizing compositions containing conformationally altered proteins are determined via a cell culture assay and an organ homogenate assay each of which is described below in detail.

### ScN2a CELL BASED ASSAY

On reading this disclosure and in particular the description provided here on particular assays, other assays will occur to those skilled in the art. The basic concept is (1) providing an acid component in an aqueous and/or alcohol carrier, (2) adding the component (not known to be active) and (3) contacting the test composition with a sample known to contain an infectious protein. After allowing time to pass (e.g. 5 minutes to 2 hours) procedures described here are used to (4) determine if the component is "active", i.e. renders the protein non-infectious. Multiple compounds can be added to the composition and tested simultaneously. If no effect is found then all of the compounds are not active. If a sterilizing effect is found the group of compounds tested can be divided in two and retested until an active compound is specifically identified. Dividing the originally tested group in any manner and retesting can be carried out any number of times.

The active component can be checked against one prion strain at a time or against multiple strains simultaneously. Some active components such as SDS will inactivate all known strains of prion while some polycationic dendrimers will inactivate only specific strains. When the active component inactivates all strains it is useful as an antiseptic and/or therapeutic. When it inactivates only a specific strain it can be used to determine the strain of infectious prion in a sample.

Efforts were made to optimize the transfection of ScN2a cells with pSPOX expression plasmids (Scott, M.R., Köhler, R., Foster, D. & Prusiner, S.B. Chimeric prion protein expression in cultured cells and transgenic mice. Protein Sci. 1, 986-997 (1992)). In connection with those effects an evaluation was made of a transfection protocol that used SuperFect reagent (QIAGEN®). It was found that epitope-tagged (MHM2) PrP^{Sc} (Scott, M.R., Köhler, R., Foster, D. & Prusiner, S.B. Chimeric prion protein expression in cultured cells and transgenic mice. Protein Sci. 1, 986-997 (1992)) could not be detected in ScN2a cells following SuperFect-mediated transfection, whereas MHM2 PrP^{Sc} was efficiently formed when a cationic liposome method for DNA delivery was used. Close scrutiny revealed that, prior to protease digestion, SuperFect-transfected samples expressed MHM2 bands, which are not seen in the background pattern of an untransfected sample. The 3F4 monoclonal antibody does not react with MoPrP but does exhibit high background staining on Western blots of mouse ScN2a cells. Increased immunostaining in the 20-30 kDa region was observed compared to the non-transfected sample. These observations led us to conclude that MHM2 PrP was successfully expressed using SuperFect transfection reagent, but that conversion of MHM2 PrP^{C} to protease-resistant MHM2 PrP^{Sc} was inhibited by SuperFect.

To investigate this apparent inhibition, a Western blot was reprobed with RO73 polyclonal antiserum to detect endogenous MoPrP^{Sc}, the presence of which is diagnostic for prion infection in ScN2a cells (Butler, D.A., et al. Scrapie-infected murine neuroblastoma cells produce protease-resistant prion proteins. J. Virol. 62, 1558-1564 (1988)). Surprisingly, it was found that the SuperFect-treated ScN2a cells no longer contained detectable quantities of MoPrP^{Sc} - also confirmed in Western blots. To investigate the mechanism by which SuperFect reduced the level of pre-existing PrP^{Sc} in chronically infected ScN2a cells, measurements were made of endogenous PrP^{Sc} in ScN2a cells exposed to various concentrations of SuperFect in the absence of plasmid DNA. The results showed that treatment with SuperFect (a branched polycation) caused the disappearance of PrP^{Sc} from ScN2a cells in a dose-dependent manner. The concentration of SuperFect required to eliminate >95% of pre-existing PrP^{Sc} with a three hour exposure was found to be about 150 µg/ml. Duration of treatment also influenced the ability of SuperFect to remove PrP^{Sc} from ScN2a cells: exposure to 150 µg/ml SuperFect for 10 min did not affect PrP^{Sc} levels, whereas 7.5 µg/ml SuperFect eliminated all detectable PrP^{Sc} with a t½= 8 h.

SuperFect, is a mixture of branched polyamines derived from heat-induced degradation of a PAMAM dendrimer (Tang, M.X., Redemann, C.T. & Szoka, F.C.J. In vitro gene delivery by degraded polyamidoamine dendrimers. Bioconjug. Chem. 7, 703-714 (1996)). Knowing this structure the ability of several other branched and unbranched polymers to eliminate PrP^{Sc} from ScN2a cells (Table 1). The branched polymers investigated include various preparations of PEI, as well as intact PAMAM and PPI dendrimers. Dendrimers are manufactured by a repetitive divergent growth technique, allowing the synthesis of successive, well-defined "generations" of homodisperse structures (Figure 1). The potency of both PAMAM and PPI dendrimers in eliminating PrP^{Sc} from ScN2a cells increased as the generation level increased. The most potent compounds with respect to eliminating PrP^{Sc} were PAMAM generation 4.0 and PPI generation 4.0, whereas PAMAM generation 1.0 showed very little ability to eliminate PrP^{Sc} (Table 1). Similarly, a high MW fraction of PEI was more potent than low MW PEI.

From the foregoing data, it is clear that for all three branched polyamines tested, increasing molecular size corresponded to an increased potency for eliminating PrP^{Sc}. To determine whether this trend was directly attributable to increased surface density of amino groups on the larger molecules, PAMAM-OH generation 4.0 was tested. This is a dendrimer that resembles PAMAM generation 4.0 except that hydroxyls replace amino groups on its surface. Unlike PAMAM generation 4.0, PAMAM-OH generation 4.0 did not cause a reduction of PrP^{Sc} levels even at the highest concentration tested (10 mg/ml), establishing that the amino groups are required for the elimination of PrP^{Sc} by PAMAM (Table 1).

In an effort to assess the contribution of the branched architecture to the clearing ability of polyamines for PrP^{Sc}, the linear molecules poly-(L)lysine and linear PEI were also tested. Both of these linear compounds were less potent than a preparation of branched PEI with similar average molecular weight (Table 1), establishing that a branched molecular architecture optimizes the ability of polyamines to eliminate PrP^{Sc}, presumably because the branched structures achieve a higher density of surface amino groups.

Kinetics of PrP^{Sc} elimination by polyamines.

The preceding results demonstrate the potent ability of branched polyamines to clear PrP^{Sc} from ScN2a cells within a few hours of treatment. The utility of these compounds to act as therapeutics for treatment of prion disease was tested by determining whether they were cytotoxic for ScN2a cells, using as criteria cell growth, morphology, and viability as measured by trypan blue staining. None of the compounds was cytotoxic to ScN2a cells after exposure for one week at concentrations up to 7.5 µg/ml. To determine whether branched polyamines can cure ScN2a cells of scrapie infection without affecting cell viability, the kinetics of prion clearance was examined in the presence of a non-cytotoxic concentration (7.5 µg/ml) of three different branched polyamines. ScN2a cells were exposed to SuperFect, PEI, or PAMAM generation 4.0 for varying periods of time. The kinetics of PrP^{Sc} elimination were assessed by Western blotting. All three compounds caused a substantial reduction in PrP^{Sc} levels after 8-16 h of treatment, and of the three compounds, PEI appeared to remove PrP^{Sc} most quickly, with a t½=4 h.

Curing neuroblastoma cells of scrapie infection.

The above results show that it is possible to reverse the accumulation of PrP^{Sc} in ScN2a cells under non-cytotoxic conditions. It was also found that extended exposure to even lower levels of the branched polyamines (1.5 µg/ml) was sufficient to eliminate PrP^{Sc}. Based on these findings, this protocol was used to determine whether the severe reduction in PrP^{Sc} levels following exposure to branched polyamines would persist after removal of the compounds. Following the exposure of ScN2a cells to a 1.5 µg/ml SuperFect for 1 week, PrP^{Sc} was reduced to <1% of the baseline level, but then increased back to ∼5% of the baseline level after 3 additional weeks in culture in the absence of polyamine. In contrast, following exposure to 1.5 µg/ml of either PEI or PAMAM generation 4.0 for 1 week, PrP^{Sc} was completely eliminated and did not return even after 3 weeks in culture without polyamines. A more intensive course of treatment with 1.8 µg/ml SuperFect for 9 d also cured ScN2a cells of scrapie infection fully, manifested by the absence of PrP^{Sc} 1 month after removal of SuperFect.

Evidence for polyamines acting within an acidic compartment.

The above results showed the potent activity of branched polyamines in rapidly clearing scrapie prions from cultured ScN2a cells. Based on these results the mechanism by which these compounds act was investigated. All of the compounds which effect removal of PrP^{Sc} from ScN2a cells are known to traffic through endosomes (Boussif, O., et al. A versatile vector for gene and oligonucleotide transfer into cells in culture and in vivo: polyethyleneimine. Proc. Natl. Acad. Sci. U.S.A. 92, 7297-7301 (1995); Haensler, J. & Szoka, F.C.J. Polyamidoamine cascade polymers mediate efficient transfection of cells in culture. Bioconjug. Chem. 4, 372-379 (1993)). Since PrP^{C} is converted into PrP^{Sc} in caveolae-like domains (CLDs) or rafts (Gorodinsky, A. & Harris, D.A. Glycolipid-anchored proteins in neuroblastoma cells form detergent-resistant complexes without caveolin. J. Cell Biol. 129, 619-627 (1995); Taraboulos, A., et al. Cholesterol depletion and modification of COOH-terminal targeting sequence of the prion protein inhibits formation of the scrapie isoform. J. Cell Biol. 129, 121-132 (1995); Vey, M., et al. Subcellular colocalization of the cellular and scrapie prion proteins in caveolae-like membranous domains. Proc. Natl. Acad. Sci. USA 93, 14945-14949 (1996); Kaneko, K., et al. COOH-terminal sequence of the cellular prion protein directs subcellular trafficking and controls conversion into the scrapie isoform. Proc. Natl. Acad. Sci. USA 94, 2333-2338 (1997)) and is then internalized through the endocytic pathway (Caughey, B., Raymond, G.J., Ernst, D. & Race, R.E. N-terminal truncation of the scrapie-associated form of PrP by lysosomal protease(s): implications regarding the site of conversion of PrP to the protease-resistant state. J. Virol. 65, 6597-6603 (1991); Borchelt, D.R., Taraboulos, A. & Prusiner, S.B. Evidence for synthesis of scrapie prion proteins in the endocytic pathway. J. Biol. Chem. 267, 16188-16199 (1992)), it was deduced that polyamines act upon PrP^{Sc} in endosomes or lysosomes. This deduction was investigated by determining the effect of pretreatment with the lysosomotropic agents chloroquine and NH₄Cl on the ability of polyamines to eliminate PrP^{Sc}. These lysosomotiopic agents alkalinize endosomes and have no effect on PrP^{Sc} levels when administered to ScN2a cells (Taraboulos, A., Raeber, AJ., Borchelt, D.R., Serban, D. & Prusiner, S.B. Synthesis and trafficking of prion proteins in cultured cells. Mol. Biol. Cell 3, 851-863 (1992)). Experimental results obtained shows that 100 µM chloroquine, but not 30 µM NH₄Cl, blocked the ability of PEI to eliminate PrP^{Sc}. Similar results were obtained with SuperFect and PAMAM, generation 4.0. Although the failure of NH₄Cl to affect PrP^{Sc} levels is not easily explained, the ability of chloroquine to attenuate the ability of branched polyamines to remove PrP^{Sc} is consistent with the notion that these agents act in endosomes or lysosomes.

### ORGAN HOMOGENATE ASSAY

The above results with cell cultures prompted investigating the possibility that in an acidic environment branched polyamines, either by indirectly interacting with PrP^{Sc} or with another cellular component, could cause PrP^{Sc} to become susceptible to hydrolases present in the endosome/lysozome. An in vitro degradation assay was developed to evaluate the effect of pH on the ability of polyamines to render PrP^{Sc} sensitive to protease. Crude homogenates of scrapie-infected mouse brain were exposed to a broad range of pH values in the presence or absence of SuperFect and then treated with proteinase K prior to Western blotting. Whereas PrP^{Sc} remained resistant to protease hydrolysis throughout the pH range (3.6-9:6) in the absence of Superfect, addition of the branched polyamine at pH 4.0 or below caused PrP^{Sc} to become almost completely degraded by protease.

Polyamine addition showed a dramatic effect on clearance in vitro which was optimized at pH 4 or less. These results show that polyamines act on PrP^{Sc} in an acidic compartment. To establish that the in vitro degradation assay is a valid approximation of the mechanism by which branched polyamines enhance the clearance of PrP^{Sc} from cultured cells, a structure activity analysis was performed with several of the compounds tested in culture cells. An excellent correlation was found between the clearance of PrP^{Sc} in cultured ScN2a cells (Table 1) and the ability to render PrP^{Sc} susceptible to protease at acidic pH in vitro. Notably, PAMAM-OH generation 4.0 failed to render PrP^{Sc} susceptible to protease, whereas PAMAM generation 4.0 and PPI, generation 4.0 exhibited an even stronger activity than Superfect in vitro, as expected from their observed potency in cultured ScN2a cells (Table 1).

### MECHANISM OF ACTION

When a pH of 4.0 or less was maintained the results show that certain branched polyamines cause the rapid elimination of PrP^{Sc} from ScN2a cells in a dose- and time-dependent manner. These compounds demonstrate a potent ability to remove prions from cultured cells at concentrations that are completely non-cytotoxic. The cells may be maintained indefinitely in culture in the presence of therapeutic levels of branched polyamines. Furthermore, when ScN2a cells were exposed to these compounds for ∼ 1 week, PrP^{Sc} was reduced to undetectable levels and remained so for at least one month after removal of the polyamine.

Clarification of the exact mechanism of PrP^{Sc} elimination by branched polyamines is an important objective. Although a number of possible scenarios exist, several possibilities may be excluded already. One possibility that was eliminated was that polyamines act by induction of chaperones such as heat shock proteins that mediate prion protein refolding because the above results show that it was possible to reproduce the phenomenon in vitro. Furthermore polyamines seem to offer advantages over other putative therapeutics that would seek to promote refolding: at very high concentrations, dimethyl sulfoxide (DMSO) and glycerol act as direct "chemical chaperones" and inhibit the formation of new PrP^{Sc} (Tatzelt, J., Prusiner, S.B. & Welch, W.J. Chemical chaperones interfere with the formation of scrapie prion protein. EMBO J. 15, 6363-6373 (1996)), but these compounds cannot reduce pre-existing PrP^{Sc} levels. Furthermore, polyamines inhibit PrP^{Sc} formation at much lower concentrations than these agents. The ability of polyamines to effect the rapid clearance of PrP^{Sc} also contrasts with the activity of other potential prion therapeutics. Sulfated polyanions may inhibit PrP^{Sc} accumulation in ScN2a cells by directly binding to PrP^{C} (Gabizon, R., Meiner, Z., Halimi, M. & Ben-Sasson, S.A. Heparin-like molecules bind differentially to prion-proteins and change their intracellular metabolic fate. J. Cell. Physiol. 157, (1993); Caughey, B., Brown, K., Raymond, G.J., Katzenstein, G.E. & Thresher, W. Binding of the protease-sensitive form of PrP (prion protein) to sulfated glycosaminoglycan and Congo red. J. Virol. 68, 2135-2141 (1991)), but because branched polyamines are able to clear pre-existing PrP^{Sc}, their mechanism of action cannot simply involve binding to PrP^{C} and inhibiting de novo synthesis.

Another possible mechanism which can be excluded is endosomal rupture. The branched polyamines which were effective in clearing PrP^{Sc} from ScN2a cells in our experiments, PEI, SuperFect and PAMAM, are also potent lysosomotropic, osmotic agents which can swell in acidic environments and rupture endosomes (Boussif, O., et al. A versatile vector for gene and oligonucleotide transfer into cells in culture and in vivo: polyethyleneimine. Proc. Natl. Acad. Sci. U.S.A. 92, 7297-7301 (1995); Haensler, J. & Szoka, F.C.J. Polyamidoamine cascade polymers mediate efficient transfection of cells in culture. Bioconjug. Chem. 4, 372-379 (1993)). This might suggest that branched polyamines clear PrP^{Sc} from ScN2a cells by rupturing endosomes and exposing PrP^{Sc} to cytosolic degradation processes. However, it is known that the lysosomotropic, endosome-rupturing agents NH₄Cl, chloroquine, and monensin do not interfere with the formation of PrP^{Sc} in ScN2a cells (Taraboulos, A., Raeber, A.J., Borchelt, D.R, Serban, D. & Prusiner, S.B. Synthesis and trafficking of prion proteins in cultured cells. Mol. Biol. Cell 3, 851-863 (1992)). Furthermore, the results also show that chloroquine interferes with the ability of branched polyamines to clear PrP^{Sc} and that polyamines can clear PrP^{Sc} in vitro at acidic pH in the absence of cell membranes. Together, these observations rule out endosome rupture as the mechanism by which branched polyamines remove PrP^{Sc}.

Without committing to any particular mechanism of action it appears likely that branched polyamines require the acidic environment of intact endosomes or lyzosomes to destroy PrP^{Sc}. The structure-activity profile of polymers tested reveals that the most active compounds possess densely packed, regularly-spaced amino groups, suggesting that these compounds may bind to a ligand which has periodically-spaced negative charges. Several scenarios remain possible. (1) Branched polyamines may bind directly to PrP^{Sc} arranged as an amyloid with exposed negatively-charged moieties and induce a conformational change under acidic conditions. (2) Treatment of PrP 27-30 with acid decreases turbidity and increases a-helical content, suggesting that such conditions might dissociate PrP^{Sc} into monomers (Safar, J., Roller, P.P., Crajdusek, D.C. & Gibbs, C.J., Jr. Scrapie amyloid (prion) protein has the conformational characteristics of an aggregated molten globule folding intermediate). It is therefore possible that polyamines bind to an equilibrium unfolding intennediate of PrP^{Sc} present under acidic conditions. (3) Alternatively, polyamines might sequester a cryptic, negatively charged component bound to PrP^{Sc} that is essential for protease resistance, but which is only released when PrP^{Sc} undergoes an acid-induced conformational change. Such a component might act as a chaperone for PrP^{Sc} inside endosomes or lysosomes. (4) Finally, another possibility is that polyamines activate an endosomal or lysosomal factor which can induce a conformational change in PrP^{Sc}. Clearly, more work will be required to determine the precise mechanism by which branched polyamines destroy PrP^{Sc}.

### GENERAL APPLICABILITY OF ASSAY

The in vitro assay described here is generally applicable in the search for compounds that effectively clear conformationally altered proteins present in food thereby preventing a number of degenerative diseases, where the accumulation of proteins seems to mediate the pathogenesis of these illnesses. By simulating lysosomes, where proteases hydrolyze proteins under acidic conditions, the in vitro brain homogenate assay is able to rapidly evaluate the efficacy of a variety of polyamines to induce degradation of PrP^{Sc}.

The in vitro assay which used scrapie infected brain homogenate to test for compounds which clear PrP^{Sc} could be modified to assay for compounds which would clear any conformationally altered protein. The assay is carried out by homogenizing the organ or tissue where the conformationally altered protein is present in the highest concentration. The pH of the homogenate is then reduced to less than 5.0 and preferably 4.0 or less. For example pancreatic tissue can be homogenized to produce an assay to test for compounds which clear amylin which is associated with type II Diabetes. Homogenized kidney could be used to test for compounds which clear β₂ - microglobulin and homogenized heart or vascular tissue used to test for compounds which clear atrial natriuretic factor. Those skilled in the art will recognize other organs and tissue types which can be homogenized to test for other compounds which clear other conformationally altered proteins.

Besides using the in vitro assay to screen for potential drugs, the compounds found via the assay such as branched polyamines provide a new tool for exploring the conversion of a protein to conformationally altered protein, e.g. PrP^{C} into PrP^{Sc}. The mechanism by which branched polyamines render PrP^{Sc} susceptible to proteolysis, remains to be established. Whether the interaction of branched polyamines with PrP^{Sc} is reversible is unknown. In addition, we do not know whether branched polyamines are able to solubilize PrP^{Sc} without irreversibly denaturing the protein. Whatever the mechanism by which branched polyamines interact with PrP^{Sc}, it is likely to be different from that found with chaotropes as well as denaturing detergents and solvents (Prusiner, S.B., Groth, D., Serban, A., Stahl, N. & Gabizon, R. Attempts to restore scrapie prion infectivity after exposure to protein denaturants. Proc. Natl. Acad. Sci. USA 90, 2793-2797 (1993)).

Using the assays described and disclosed here certain specific branched polyamines have been found which mediate the clearance of PrP^{Sc} from cultured cells under non-cytotoxic conditions. These compounds offer the intriguing possibility of being added to a wide range of low pH food products to neutralize conformational altered proteins present. Since the compounds found act by stimulating normal cellular pathways of protein degradation to destroy PrP^{Sc}, this class of compounds would also likely be of value in the treatment of other degenerative and hereditary disorders where abnormally folded, wild-type or mutant proteins accumulate. Such an approach may find merit in developing an effective therapeutics for one or more of the common, degenerative illnesses including Alzheimer's disease, Parkinson's disease, amyotrophic lateral sclerosis, frontotemporal dementia, adult onset diabetes mellitus and the amyloidoses (Beyreuther, K. & Masters, C.L. Serpents on the road to dementia and death. Accumulating evidence from several studies points to the normal function of presenilin 1 and suggests how the mutant protein contributes to deposition of amyloid plaques in Alzheimer's disease. Nature Medicine 3, 723-725 (1997); Masters, C.L. & Beyreuther, K. Alzheimer's disease. BMJ 316, 446-448 (1998); Selkoe, D.J. The cell biology of beta-amyloid precursor protein and presenilin in Alzheimer's disease. Trends in Cell Biol. 8, 447-453 (1998); Selkoe, D.J. Translating cell biology into therapeutic advances in Alzheimer's disease. Nature 399, A23-31 (1999); Wong, P.C., et al. An adverse property of a familial ALS-linked SOD1 mutation causes motor neuron disease characterized by vacuolar degeneration of mitochondria. Neuron 14, 1105-1116 (1995), Spillantini, M.G., Crowther, R.A., Jakes, R, Hasegawa, M. & Goedert, M. a-Synuclein in filamentous inclusions of Lewy bodies from Parkinson's disease and dementia with Lewy bodies. Proc. Natl. Acad. Sci. USA 95, 6469-6473 (1998); Hutton, M., et al. Association of missense and 5'-splice-site mutations in tau with the inherited dementia FTDP-17. Nature 393, 702-705 (1998); Stone, M.J. Amyloidosis: a final common pathway for protein deposition in tissues. Blood 75, 531-545 (1990)). Whether branched polyamines might also prove efficacious in a variety of inherited disorders where the accumulation of abnormal proteins is a hallmark of the illness remains to be established; these genetic maladies include heritable forms of prion disease, Alzheimer's disease, Parkinson's disease, amyotrophic lateral sclerosis, frontotemporal dementia, Pick's disease and amyloidosis, as well as the triplet repeat diseases including Huntington's disease, spinal cerebellar ataxias and myotonic dystrophy (Fu, Y.-H., et al. An unstable triplet repeat in a gene related to myotonic muscular dystrophy. Science 255, 1256-1259 (1992); Group, T.H.s.D.C.R. A novel gene containing a trinucleotide repeat that is expanded and unstable on Huntington's disease chromosomes. Cell 72, 971-983 (1993)). Compounds identified via the above-described such as branched polyamines will find utility in preventing or delaying the onset of these genetic diseases where carriers can often be identified decades in advance of detectable neurologic or systemic dysfunction.

Several dendritic polycations, including the starburst dendrimers Superfect™ (QIAGEN®, Valencia, CA), polyamidoamide (PAMAM), and the hyperbranched polycation polyethyleneimine (PEI), were surprisingly found to eliminate PrP^{Sc} from cultured scrapie-infected neuroblastoma cells. These highly-branched, polycationic compounds provide a novel class of therapeutic agents to combat prion diseases and other degenerative disease including the amyloidoses. The removal of PrP^{Sc} is dependent on both the concentration of dendritic polymer and length of exposure. Dendritic polymers were able to clear PrP^{Sc} at concentrations which were not cytotoxic. Repeated exposures to heat-degraded starburst PAMAM dendrimer or PEI caused a dramatic reduction in PrP^{Sc} levels which persisted for a month even after removal of the compound. Dendritic polycations did not appear to destroy purified PrP^{Sc} in vitro, and therefore may act through a generalized mechanism. Dendritic polycations represent a class of compounds which can be used as therapeutic agents in prion diseases and other disorders involving insoluble protein deposits, such as the amyloidoses.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Unless indicated otherwise, parts are parts by weight, molecular weight is weight average molecular weight, temperature is in degrees Centigrade, and pressure is at or near atmospheric.

### METHODS AND MATERIALS

Chemicals. All compounds were purchased from Sigma-Aldrich. All test compounds were dissolved in water at stock concentration of 3 mg/ml and filtered through a Millipore 0.22 m m filter.

Cultured cells. Stock cultures of ScN2a cells were maintained in MEM with 10% FBS, 10% Glutamax (Gibco BRL), 100 U penicillin, and 100 mg/ml streptomycin (supplemented DME). Immediately prior to addition of test compounds, the dishes were washed twice with fresh supplemented DME media. After exposure to test compounds, dishes were drained of media and cells were harvested by lysis in 0.25-1 ml 20 mM Tris pH 8.0 containing 100 mM NaCl, 0.5% NP-40, and 0.5% sodium deoxycholate to obtain a total protein concentration of 1 mg/ml measured by the BCA assay. Nuclei were removed froin the lysate by centrifugation at 2000 rpm for 5 min. For samples not treated with proteinase K, 40 µl of whole lysate (representing 40 µg total protein) was mixed with an equal volume of 2x SDS reducing sample buffer. For proteinase K digestion, 20 µg/ml proteinase K (Boehringer Mannheim) (total protein:enzyme ratio = 50:1) was added, and the sample was incubated for 1 h at 37°C. Proteolytic digestion was terminated by the addition of Pefabloc to a final concentration of 5 mM. One ml samples were centrifuged at 100,000 x g for 1 hat 4°C, the supernatants were discarded, and the pellets were resuspended in 80 µl of reducing SDS sample buffer for SDS-PAGE.

Brain homogenates. Brain homogenates from RML scrapie-affected CD-1 mice (10% (w/v) in sterile water) were prepared by repeated extrusion through syringe needles of successively smaller size, from 18 to 22 gauge. Nuclei and debris were removed by centrifugation at 1000 x g for 5 min. The bicinchnoninic acid (BCA) protein assay (Pierce) was used to determine protein concentration. Homogenates were adjusted to 1 mg/ml protein in 1% NP-40. For reactions, 0.5 ml homogenate was incubated with 25 ml 1.0 M buffer (sodium acetate for pH 3-6 and Tris acetate for pH 7-10) plus or minus 10 ml of polyamine stock solution (3 mg/ml) for 2 h at 37°C with constant shaking. The final pH value of each sample was measured directly with a calibrated pH electrode (Radiometer Copenhagen). Following incubation, each sample was neutralized with an equal volume 0.2 M HEPES pH 7.5 containing 0.3 M NaCl and 4% Sarkosyl. Proteinase K was added to achieve a final concentration of 20 µg/ml, and samples were incubated for 1 h at 37°C. Proteolytic digestion was terminated by the addition of Pefabloc to a final concentration of 5 µM. Ten µl of digested brain homogenate was mixed with equal volume 2x SDS sample buffer and analyzed by SDS-PAGE followed by Western blotting.

Western blotting. Following electrophoresis, Western blotting was performed as previously described (Scott, M., et al. Transgenic mice expressing hamster prion protein produce species-specific scrapie infectivity and amyloid plaques. Cell 59, 847-857 (1989)). Samples were boiled for 5 min and cleared by centrifugation for 1 min at 14,000 rpm in aBeckman ultrafuge. SDS-PAGE was carried out in 1.5 mm, 12% polyacrylamide gels(Laemmli, U.K. Cleavage of structural proteins during the assembly of the head of bacteriophage T-4. Nature 227, 680-685 (1970)). Membranes were blocked with 5% non-fat milk protein in PBST (calcium- and magnesium-free PBS plus 0.1% Tween 20) for 1 h at room temperature. Blocked membranes were incubated with primary RO73 polyclonal antibody (to detect MoPrP) (Serban, D., Taraboulos, A., DeArmond, S.J. & Prusines, S.B. Rapid detection of Creutzfeldt-Jakob disease and scrapie prion proteins. Neurology 40, 110-117 (1990)) or 3F4 monoclonal antibody (to detect MHM2 PrP) (Kascsak, R.J., et al. Mouse polyclonal and monoclonal antibody to scrapie-associated fibril proteins. J. Virol. 61, 3688-3693 (1987)) at 1:5000 dilution in PBST overnight at 4°C. Following incubation with primary antibody, membranes were washed 3 x 10 min in PBST, incubated with horseradish peroxidase-labeled secondary antibody (Amersham Life Sciences) diluted 1:5000 in PBST for 30 to 60 min at 4°C and washed again for 3 x 10 min in PBST. After chemiluminescent development with ECL reagent (Amersham) for 1 min, blots were sealed in plastic covers and exposed to ECL Hypermax film (Amersham). Films were processed automatically in a Konica film processor.

### EXAMPLE 1

Results provided here show that acidic conditions (<pH 5) enhance the ability of detergents (e.g. SDS) to denature PrP^{Sc} and destroy prion infectivity. Specific results demonstrate that acidic conditions can be used to formulate effective prion disinfectants emphasizing the importance of acidic conditions.

### THE EFFECTIVENESS OF 1% SDS as pH 3.4 to ELIMINATE PrP^{Sc} at 5 MINUTES OR 2 HOURS

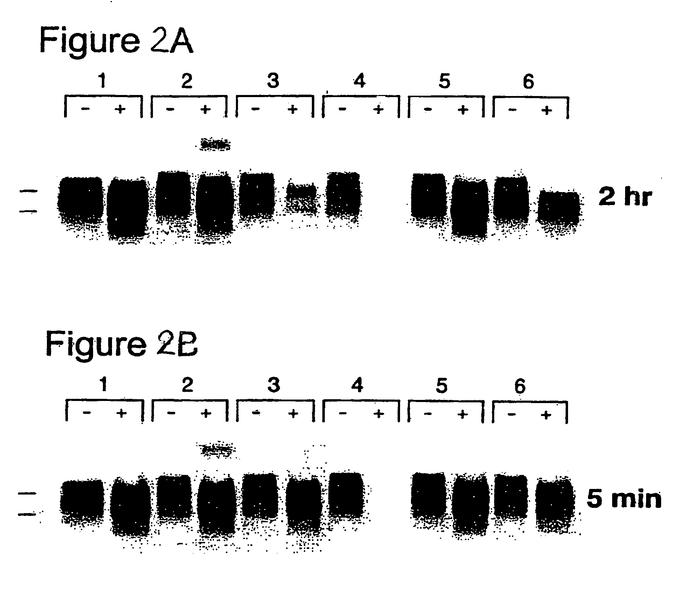

A 2% homogenate of scrapie Sc237 brain in water was prepared by repeated extrusion through a 22 G needle. Nuclei were removed by centrifugation for 5 min at 1000 rpm. The clarified homogenate was diluted 2-fold and incubated for (Top panel of Figure 2A) 2 hrs or (Bottom panel of Figure 2B) 5 min at 37°C under the following conditions:
1. 1% NP40, 50 mM sodium acetate pH 7.0
2. 1% NP40, 0.5% acetic acid pH 3.4
3. 1% SDS, 50 mM sodium acetate pH 7.0
4. 1% SDS, 0.5% acetic acid pH 3.4
5. 1% Sarkosyl, 50 mM sodium acetate pH 7.0
6. 1% Sarkosyl, 0.5% acetic acid pH 3.4

Plus (+) lanes indicate samples subjected to limited proteolysis with 20 µg/ml proteinase K for 1 hr at 37°C, Minus (-) lanes indicate samples not subjected to proteolysis. All samples were boiled in SDS sample buffer for 5 min prior to SDS polyacrylamide gel eletrophoresis. Following transfer toMillipore Immobilon transfer membrane, development of the immunoblot was performed with d13 primary Fab.

### THE EFFECT OF TEMPERATURE ON THE ABILITY OF 1% SDS AT pH 3.4 TO ELIMINATE PrP^{Sc}

A 2% homogenate of scrapie Sc237 brain in water was prepared by repeated extrusion through a 22 G needle. Nuclei were removed by centrifugation for 5 min at 1000 rpm. The clarified homogenate was diluted 2-fold and incubated for 5 min under the following conditions:
1. 1% SDS, 0.5% acetic acid pH 3.4 at 4°C
2. 1% SDS, 0.5% acetic acid pH 3.4 at 20°C
3. 1% SDS, 0.5% acetic acid pH 3.4 at 37°C
4. 1% NP40, 0.5% acetic acid pH 3.4 at 20°C

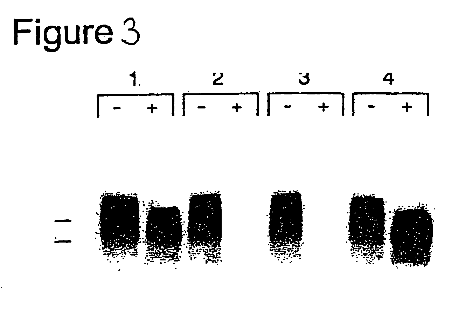

Plus (+) lanes of Figure 3 indicate samples subjected to limited proteolysis with 20 µg/ml proteinase K for 1 hr at 37°C. Minus (-) lanes indicate samples not subjected to proteolysis. All samples were boiled in SDS samples buffer for 5 min prior to SDS polyacrylamide gel electrophoresis. Following transfer to Millipore Immobilon transfer membrane, development of the immunoblot was performed with d13 primary Fab.

### EXAMPLE 2

### SDS/ACETIC ACID FORMULATION

Samples of 1% Syrian hamster brain homogenate containing 10⁷ LD₅₀ units prion infectivity/ml were incubated with either 50 mM Tris acetate pH 7.0 or 0.5% acetic acid in the presence of either 1% NP-40 or 1% SDS for 2 h at 37°C. Following incubation, each sample was inoculated intracerebrally into 8 separate Syrian hamsters for a scrapie incubation time assay. The results are shown in the table below:

| sample | LD₅₀/ml |
|---|---|
| 1% NP40, 50 mM Tris acetate pH 7.0 | 10⁷ |
| 1% NP40, 5% acetic acid, pH 3.6 | 10⁷ |
| 1% SDS, 50 mM Tris acetate pH 7.0 | 10⁵ |
| 1% SDS, 5% acetic acid, pH 3.6 | <10² |

The above results clearly demonstrate that a formulation comprised of approximately 1% SDS and approximately 0.5% acetic acid is effective in inactivating prions. Such a formulation could provide for an extremely valuable commercial formulation due to the ready availability of both acetic acid and SDS. However, those skilled in the art will recognize that other effective acids and effective detergents with structure similar to SDS could be formulated to obtain the same or similar results. With respect to the acid component what is important is to create a formulation which keeps the pH of the formulation acidic and preferably below 4.0. The detergent composition need not be SDS. For example, the sodium component of the detergent could be any cation such as calcium, lithium, potassium, magnesium etc. Further, the sulfate component could be substituted with chemically equivalent moieties. Sodium dodecyl sulfate includes a hydrocarbon component with eleven CH₂ groups terminated by a CH₃ group. Various other alkyl groups such as other straight chained, branched or cyclic groups could be utilized. The alkyl moiety could contain from 2 to 40 carbons and more preferably contains approximately 12 carbon atoms ± 6 carbon atoms. The formulation can be added to appropriate solvents, in appropriate concentrations. Further, the concentration of the formulation can be changed immediately prior to use and thus sold at a highly concentrated formulation or sold in a concentration ready for use without dilution with a solvent such as water or alcohol. Still further, as indicated above the formulations of the invention can be supplemented with appropriate antibacterial and/or antiviral components as well as components which inactivate other pathogens including parasites so that the final formulation is effective in killing or inactivating a wide range of infectious components.

### EXAMPLE 3

### EFFECT OF DETERGENT AND pH ON PROTEASE-RESISTANT PrP^{Sc}

Samples of 1% Sc237-infected SHa brain homogenate were incubated for 15 min at 37°C with detergent at a range pH values as indicated. Fifty millimolar sodium acetate buffers were used to maintain pH values 3-6, and 50 mM Tris acetate buffers were used to maintain pH values 7-10. The final pH value of each sample denoted above the corresponding lanes was measured directly with a calibrated pH electrode (Radiometer Copenhagen). All samples were neutralized by addition of equal volume 4% Sarkosyl, 100 mM HEPES pH 7.5, 200 mM NaCl and subjected to limited proteolysis with 20 µg/ml proteinase K for 1 h at 37°C. Apparent molecular weights based on migration of protein standards are 30 and 27 kDa. All samples were neutralized by addition of equal volume 4% Sarkosyl, 100 mM HEPES pH 7.5, 200 mM NaCl. Minus (-) symbol denotes undigested, control sample and plus (+) symbol designates sample subjected to limited proteolysis with 20 µg/ml proteinase K for 1 h at 37°C. Apparent molecular weights based on migration of protein standards are 30 and 27 kDa. Figure 3 shows that SDS denatures PrPSc at pH <5 or pH ≥ 10.

### EXAMPLE 4

### CHARACTERIZATION OF PrP^{Sc} DENATURATION MEDIATED BY ACIDIC SDS

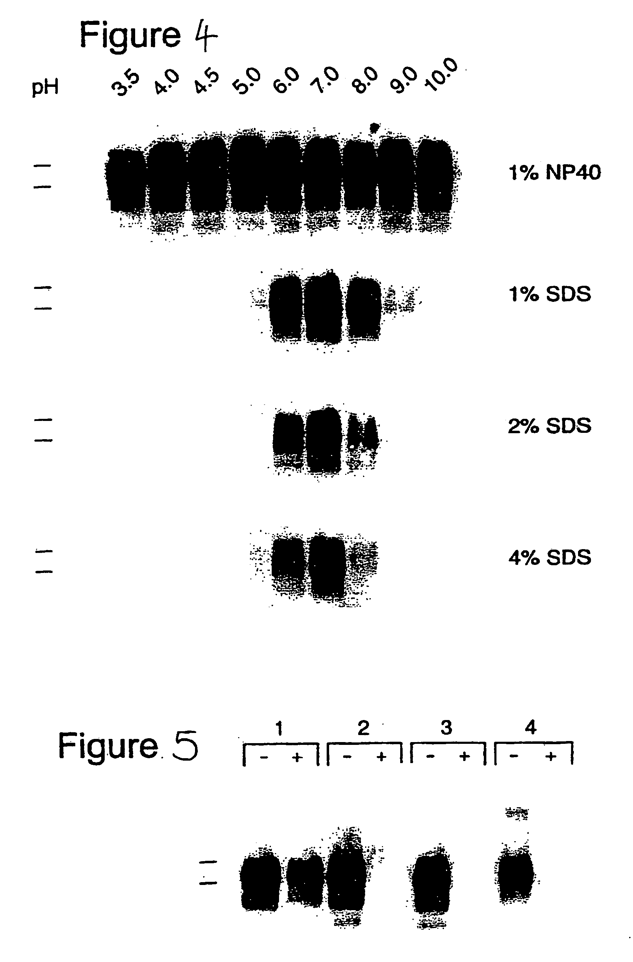

Samples of 1% Sc237-infected SHa brain homogenate were incubated for 15 min at 37°C in 1% SDS plus (1) 50 mM Tris acetate pH 7.0, (2) 50 mM sodium acetate pH 3.6, (3) 50 mM glycine pH 3.7, and (4) 0.2% peracetic acid, pH 3.4. Following incubation, an equal volume of 4% Sarkosyl, 100 mM HEPES pH 7.5, 200 mM NaCl was added to neutralize each sample. Minus (-) symbol denotes undigested, control sample and plus (+) symbol designates sample subjected to limited proteolysis with 20 µg/ml proteinase K for 1 h at 37°C. Apparent molecular weights based on migration of protein standards are 30 and 27 kDa. Figure 4 shows that SDS denatures PrPSc under acidic conditions in different acidic buffers, including peracetic acid (a commonly used hospital disinfectant).

## Claims

1. A method of treating a material to inactivate infectious prions, comprising the steps of:
contacting the material with a composition, comprising:
a solvent;
an acid component **characterised by** and present in a molarity so as to maintain the pH of the composition in a range of from 2.5 to 4.5; and
a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether; and
allowing the composition to remain in contact with the material which eliminates the infectivity of the infectious prions.

2. The method of claim 1, wherein the solvent is selected from water, alcohol and a mixture thereof.

3. The method of claim 1 or 2, wherein the acid component is selected from acetic acid and peracetic acid.

4. The method of claim 1 or 2, wherein the acid component is selected from a sulfuric acid, a hydrochloric acid, a nitric acid and a carboxylic acid and wherein the detergent is **characterised by** reducing infectivity of the infectious prions when the composition is brought into contact with the infectious prions for one hour or less at a temperature of 40°C or less at a pressure of one atmosphere.

5. The method of claim 4, wherein the detergent is sodium dodecyl sulphate (SDS).

6. The method of any one of the preceding claims, wherein the solvent is selected from water, alcohol and a mixture thereof present in an amount of 1% to 99.99% by weight and wherein the detergent is present in an amount of 0.001% to 10% by weight.

7. The method of claim 6, wherein the composition further comprises:
an antibacterial compound in an amount of 0.1% to 20% by weight;
an antiviral compound in an amount of 0.1% to 20% by weight; and
an antifungal compound in an amount of 0.1% to 20% by weight;
wherein the detergent is present in an amount of 1% to 10% by weight.

8. The method of any one of the preceding claims, wherein the material is selected from a pharmaceutical gel capsule, a gel coated tablet, a blood extender or blood replacement solution, a surgical implant, a bandage, a suture, a dental implement, a dental sponge, a surgical sponge, a candy containing a gelatin, a marshmallow, or a mint doughnut glaze, fruit juice, wine, beer, sour cream, yogurt, cottage cheese, ice cream, margarine and chewing gum.

9. A method of forming a gelatin capsule, comprising:
extracting hooves from an ungulate;
treating the hooves so as to create a gelatin material;
treating the gelatin with an acid of a type and in an amount as to reduce the pH of the gelatin to 4.0 or less;
adding to the gelatin a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether, which eliminates the infectivity of infectious prions.

10. A gelatin capsule produced by the method of claim 9.

11. The gelatin capsule as claimed in claim 10, having therein a compound selected from a pharmaceutically active drug, a vitamin and a nutriceutical.

12. A method of sterilising an object, comprising the step of:
contacting the object with an antiseptic composition comprised of
a solvent;
an acid component **characterised** and present in a molarity so as to maintain the pH of the composition in a range of from 2.5 to 4.5; and
a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether, which eliminates the infectivity of infectious protein.

13. The method of claim 12, wherein the object is selected from a surgical instrument, a diagnostic instrument, a colonoscope, a sigmoidoscope, a bronchoscope, a gastroscope, a dental instrument, a catheter, an operating room, a cell culture, a chromotographic column and gelatin material obtained from a cow.

14. The method of claim 12 or 13, wherein the acid component is selected from acetic acid and peracetic acid.

15. The method of claim 12, wherein the detergent is sodium dodecyl sulphate (SDS).

16. A method of producing a pharmaceutically active protein in sterile form, comprising:
culturing cells designed to express a protein of interest;
extracting the protein of interest; and
contacting the protein of interest with an antiseptic composition comprising
a solvent;
an acid component **characterised by** and present in a molarity so as to maintain the pH of the composition in a range of from 2.5 to 4.5; and
a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether, which eliminates the infectivity of infectious protein.

17. The method of claim 16 wherein the infectious protein is a prion.

18. An antiseptic composition comprising:
a solvent selected from water, alcohol and a mixture thereof in an amount of 1% to 99.99% by weight;
an acid component **characterised by** and present in a molarity so as to maintain the pH of the composition in a range of from 2.5 to 4.5;
a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether, in an amount of 1% to 10% by weight;
an antibacterial compound in an amount of 0.1% to 20% by weight;
an antiviral compound in an amount of 0.1% to 20% by weight; and
an antifungal compound in an amount of 0.1% to 20% by weight.

19. The composition of claim 18, wherein the acid component is of a type chosen from a sulfuric acid, a hydrochloric acid, a nitric acid and a carboxylic acid.

20. Use of a composition comprising:
a solvent;
an acid component **characterised by** and present in a molarity so as to maintain the pH of the composition in a range of from 2.5 to 4.5; and
a detergent chosen from sodium dodecyl sulfate (SDS), lithium dodecyl sulfate, potassium dodecyl sulfate, sodium cholate, sodium deoxycholate, octylglucoside, dodecyldimethylamine oxide, 3-[(3-cholamidopropyl) dimethylammonio]-1-propanesulfonate (CHAPS), dodecyltriethylammonium bromide (DTAB), cetyltrimethylammonium bromide (CTAB) and polyoxyethylene-p-isooctylphenyl ether;
for treating a material to eliminate the infectivity of infectious prions.

21. The method of claim 1, 9, 12 or 16, wherein the polyoxyethylene-p-isooctylphenyl ether is Triton X-20, Triton X-100 or Triton X-114.

22. The composition of claim 18, wherein the polyoxyethylene-p-isooctylphenyl ether is Triton X-20, Triton X-100 or Triton X-114.

23. The use of claim 20, wherein the polyoxyethylene-p-isooctylphenyl ether is Triton X-20, Triton X-100 or Triton X-114.

## Patentansprüche

1. Verfahren zum Behandeln eines Materials, um infektiöse Prionen zu inaktivieren, umfassend die Schritte:
Inkontaktbringen des Materials mit einer Zusammensetzung, umfassend:
ein Lösungsmittel
einen Säurebestandteil, **gekennzeichnet durch** eine und vorliegend in einer Molarität, bei welcher der pH-Wert der Zusammensetzung in einem Bereich von 2,5 bis 4,5 gehalten wird, und
ein Detergens, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether, und
Inkontaktlassen der Zusammensetzung mit dem Material, das die Infektiösität der infektiösen Prionen eliminiert.

2. Verfahren nach Anspruch 1, wobei das Lösungsmittel ausgewählt ist aus Wasser, Alkohol und einem Gemisch davon.

3. Verfahren nach Anspruch 1 oder 2, wobei der Säurebestandteil ausgewählt ist aus Essigsäure und Peressigsäure.

4. Verfahren nach Anspruch 1 oder 2, wobei der Säurebestandteil ausgewählt ist aus Schwefelsäure, Salzsäure, Salpetersäure und einer Carbonsäure und wobei das Detergens durch Reduzieren der Infektiösität der infektiösen Prionen **gekennzeichnet** ist, wenn die Zusammensetzung mit den infektiösen Prionen über eine Dauer von einer Stunde oder weniger bei einer Temperatur von 40°C oder weniger bei einem Druck von einer Atmosphäre in Kontakt gebracht wird.

5. Verfahren nach Anspruch 4, wobei das Detergens Natriumdodecylsulfat (SDS) ist.

6. Verfahren nach einem der vorangehenden Ansprüche, wobei das Lösungsmittel ausgewählt ist aus Wasser, Alkohol und einem Gemisch davon, das in einer Menge von 1 bis 99 Gew.-% vorliegt, und wobei das Detergens in einer Menge von 0,001 bis 10 Gew.-% vorliegt.

7. Verfahren nach Anspruch 6, wobei die Zusammensetzung ferner Folgendes umfasst:
eine antibakterielle Verbindung in einer Menge von 0,1 bis 20 Gew.-%,
eine antivirale Verbindung in einer Menge von 0,1 bis 20 Gew.-% und
eine fungizide Verbindung in einer Menge von 1 bis 10 Gew.-%.

8. Verfahren nach einem der vorangehenden Ansprüche, wobei das Material ausgewählt ist aus einer pharmazeutischen Gelkapsel, einer gelbeschichteten Tablette, einem Blutstreckmittel oder einer Blutersatzlösung, einem chirurgischen Implantat, einer Bandage, einem Zahnimplantat, einem Dentaltupfer, einem chirurgischen Tupfer, einem Gelatine enthaltenden Bonbon, einem Marshmallow, Doughnutglasur, Fruchtsaft, Wein, Bier, saurer Sahne, Yoghurt, Hüttenkäse, Eiscreme, Margarine und Kaugummi.

9. Verfahren zum Formen einer Gelatinekapsel, umfassend
Extrahieren von Hufen von Huftieren,
Behandeln der Hufe so, dass ein Gelatinematerial gebildet wird,
Behandeln der Gelatine mit einer Säure eines solchen Typs und in einer solchen Menge, dass der pH-Wert der Gelatine 4,0 oder weniger beträgt, Zugabe zur Gelatine eines Detergenzes, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether, das die Infektiösität von infektiösen Prionen eliminiert.

10. Gelatinekapsel, hergestellt durch das Verfahren nach Anspruch 9.

11. Gelatinekapsel nach Anspruch 10, die darin eine Verbindung, ausgewählt aus einem pharmazeutisch aktiven Arzneimittel, einem Vitamin und einem Nährstoff, aufweist.

12. Verfahren zum Sterilisieren eines Gegenstands, umfassend den Schritt Inkontaktbringen des Gegenstands mit einer antiseptischen Zusammensetzung, zusammengesetzt aus
einem Lösungsmittel
einem Säurebestandteil, **gekennzeichnet durch** eine und vorliegend in einer Molarität, bei welcher der pH-Wert der Zusammensetzung in einem Bereich von 2,5 bis 4,5 gehalten wird, und
einem Detergens, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether, das die Infektiösität der infektiösen Prionen eliminiert.

13. Verfahren nach Anspruch 12, wobei der Gegenstand ausgewählt ist aus einem chirurgischen Instrument, einem diagnostischen Instrument, einem Kolonoskop, einem Sigmoidoskop, einem Bronchoskop, einem Gastroskop, einem Dentalinstrument, einem Katheter, einem Operationssaal, einer Zellkultur, einer Chromatograficsäule und einem von einer Kuh erhaltenen Gelatinematerial.

14. Verfahren nach Anspruch 12 oder 13, wobei der Säurebestandteil ausgewählt ist aus Essigsäure und Peressigsäure.

15. Verfahren nach Anspruch 12, wobei das Detergens Natriumdodecylsulfat (SDS) ist.

16. Verfahren zum Herstellen eines pharmazeutisch aktiven Proteins in steriler Form, umfassend
Züchten von Zellen, die zum Exprimieren eines Proteins von Interesse bestimmt sind,
Extrahieren des Proteins von Interesse und
Inkontaktbringen des Proteins von Interesse mit einer antiseptischen Zusammensetzung, umfassend
ein Lösungsmittel
einen Säurebestandteil, **gekennzeichnet durch** eine und vorliegend in einer Molarität, bei welcher der pH-Wert der Zusammensetzung in einem Bereich von 2,5 bis 4,5 gehalten wird, und
ein Detergens, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether, das die Infektiösität der infektiösen Prionen eliminiert.

17. Verfahren nach Anspruch 16, wobei das infektiöse Protein ein Prion ist.

18. Antiseptische Zusammensetzung, umfassend
ein Lösungsmittel, ausgewählt aus Wasser, Alkohol und einem Gemisch davon in einer Menge von 1 bis 99,99 Gew.-%,
einen Säurebestandteil, **gekennzeichnet durch** eine und vorliegend in einer Molarität, bei welcher der pH-Wert der Zusammensetzung in einem Bereich von 2,5 bis 4,5 gehalten wird, und
ein Detergens, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether,
eine antibakterielle Verbindung in einer Menge von 0,1 bis 20 Gew.-%,
eine antivirale Verbindung in einer Menge von 0,1 bis 20 Gew.-% und
eine fungizide Verbindung in einer Menge von 1 bis 20 Gew.-%.

19. Zusammensetzung nach Anspruch 18, wobei der Säurebestandteil von einem Typ, ausgewählt aus Schwefelsäure, Salzsäure, Salpetersäure und Carbonsäure, ist.

20. Verwendung einer Zusammensetzung, umfassend
ein Lösungsmittel,
einen Säurebestandteil, **gekennzeichnet durch** eine und vorliegend in einer Molarität, bei welcher der pH-Wert der Zusammensetzung in einem Bereich von 2,5 bis 4,5 gehalten wird, und
ein Detergens, ausgewählt aus Natriumdodecylsulfat (SDS), Lithiumdodecylsulfat, Kaliumdodecylsulfat, Natriumcholat, Natriumdeoxycholat, Octylglucosid, Dodecyldimethylaminoxid, 3-[(3-Cholamidopropyl)dimethylammonio]-1-propansulfonat (CHAPS), Dodecyltriethylammoniumbromid (DTAB), Cetyltrimethylammoniumbromid (CTAB) und Polyoxyethylen-p-isooctylphenylether,
eine antibakterielle Verbindung in einer Menge von 0,1 bis 20 Gew.-%.
zur Behandlung eines Materials zum Eliminieren der Infektösität von infektiösen Prionen.

21. Verfahren nach Anspruch 1, 9, 12 oder 16, wobei der Polyoxyethylen-p-isooctylether Triton X-20, Triton X-100 oder Triton X-114 ist.

22. Zusammensetzung nach Anspruch 18, wobei der Polyoxyethylen-p-isooctylether Triton X-20, Triton X-100 oder Triton X-114 ist.

23. Zusammensetzung nach Anspruch 20, wobei der Polyoxyethylen-p-isooctylether Triton X-20, Triton X-100 oder Triton X-114 ist.

## Revendications

1. Procédé de traitement de matériaux en vue d'inactiver des prions infectieux, qui comporte les étapes suivantes :
- mettre un matériau en contact avec une composition qui comprend :
- un solvant,
- un composant acide, de nature telle et présent en une molarité telle que le pH de la composition se maintient dans l'intervalle allant de 2,5 à 4,5,
- et un détergent choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé ;
- et laisser cette composition demeurer en contact avec le matériau, ce qui permet d'annihiler l'infectiosité des prions infectieux.

2. Procédé conforme à la revendication 1, dans lequel le solvant est choisi parmi de l'eau, un alcool, et un mélange d'eau et d'un alcool.

3. Procédé conforme à la revendication 1 ou 2, dans lequel le composant acide est choisi parmi l'acide acétique et l'acide peracétique.

4. Procédé conforme à la revendication 1 ou 2, dans lequel le composant acide est choisi parmi l'acide sulfurique, l'acide chlorhydrique, l'acide nitrique et un acide carboxylique, et le détergent est **caractérisé en ce qu'**il réduit l'infectiosité des prions infectieux quand la composition est laissée en contact avec les prions infectieux pendant 1 heure ou moins, à une température inférieure ou égale à 40 °C et sous une pression de 1 atm.

5. Procédé conforme à la revendication 4, dans lequel le détergent est du dodécylsulfate de sodium (SDS).

6. Procédé conforme à l'une des revendications précédentes, dans lequel le solvant, choisi parmi de l'eau, un alcool, et un mélange d'eau et d'un alcool, est présent en une proportion pondérale de 1 à 99,99 %, et le détergent est présent en une proportion pondérale de 0,001 à 10 %.

7. Procédé conforme à la revendication 6, dans lequel la composition contient en outre :
- un composé antibactérien, présent en une proportion pondérale de 0,1 à 20 %,
- un composé antiviral, présent en une proportion pondérale de 0,1 à 20 %,
- et un composé antifongique, présent en une proportion pondérale de 0,1 à 20 %,
et dans lequel le détergent est présent en une proportion pondérale de 1 à 10 %.

8. Procédé conforme à l'une des revendications précédentes, dans lequel le matériau est choisi parmi une capsule de gélatine pharmaceutique, un comprimé enrobé de gel, une solution de remplacement du sang ou de dilution du sang, un implant chirurgical, un bandage, un fil à sutures, un implant dentaire, une éponge dentaire, une éponge chirurgicale, un bonbon gélatineux, un marshmallow, un bonbon à la menthe, un glaçage à beignets, un jus de fruits, du vin, de la bière, de la crème fermentée, du yaourt, du fromage blanc, de la crème glacée, de la margarine ou de la gomme à mâcher.

9. Procédé de fabrication de capsules de gélatine, qui comporte les étapes suivantes ::
- prélever des sabots d'ongulés ;
- traiter ces sabots de manière à obtenir un matériau gélatineux ;
- traiter cette gélatine avec un acide, de nature telle et employé en une quantité telle que le pH de la gélatine est réduit à une valeur au plus égale à 4,0 ;
- et ajouter à cette gélatine un détergent, choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé, qui annihile l'infectiosité des prions infectieux.

10. Capsule de gélatine produite suivant un procédé conforme à la revendication 9.

11. Capsule de gélatine conforme à la revendication 10, qui con-tient un composé choisi parmi un produit doté d'une activité pharmaceuti-que, une vitamine et un produit nutraceutique.

12. Procédé de stérilisation d'objets, qui comporte l'étape suivante :
- mettre un objet en contact avec une composition antiseptique qui comprend :
- un solvant,
- un composant acide, de nature telle et présent en une molarité telle que le pH de la composition se maintient dans l'intervalle allant de 2,5 à 4,5,
- et un détergent, choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé, qui annihile l'infectiosité des prions infectieux.

13. Procédé conforme à la revendication 12, dans lequel l'objet est choisi parmi un instrument chirurgical, un instrument de diagnostic, un colonoscope, un sigmoïdoscope, un bronchoscope, un gastroscope, un instrument de chirurgie dentaire, un cathéter, une salle d'opération, une culture cellulaire, une colonne chromatographique, et un matériau gélatineux obtenu à partir de bovin.

14. Procédé conforme à la revendication 12 ou 13, dans lequel le composant acide est choisi parmi l'acide acétique et l'acide peracétique.

15. Procédé conforme à la revendication 12, dans lequel le détergent est du dodécylsulfate de sodium (SDS).

16. Procédé de production d'une protéine pharmacologiquement active, sous forme stérile, lequel procédé comporte les étapes suivantes :
- cultiver des cellules conçues pour exprimer une protéine intéressante ;
- extraire la protéine intéressante ;
- et mettre cette protéine intéressante en contact avec une composition antiseptique qui comprend :
- un solvant,
- un composant acide, de nature telle et présent en une molarité telle que le pH de la composition se maintient dans l'intervalle allant de 2,5 à 4,5,
- et un détergent, choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé, qui annihile l'infectiosité d'une protéine infectieuse.

17. Procédé conforme à la revendication 16, dans lequel la protéine infectieuse est un prion.

18. Composition antiseptique comprenant :
- un solvant, choisi parmi de l'eau, un alcool, et un mélange d'eau et d'un alcool, et présent en une proportion pondérale de 1 à 99,99 %,
- un composant acide, de nature telle et présent en une molarité telle que le pH de la composition se maintient dans l'intervalle allant de 2,5 à 4,5,
- un détergent choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé, et présent en une proportion pondérale de 1 à 10 % ;
- un composé antibactérien, présent en une proportion pondérale de 0,1 à 20 %,
- un composé antiviral, présent en une proportion pondérale de 0,1 à 20 %,
- et un composé antifongique, présent en une proportion pondérale de 0,1 à 20 %.

19. Composition conforme à la revendication 18, dans laquelle le composant acide est choisi parmi l'acide sulfurique, l'acide chlorhydrique, l'acide nitrique et un acide carboxylique.

20. Emploi d'une composition comprenant :
- un solvant
- un composant acide, de nature telle et présent en une molarité telle que le pH de la composition se maintient dans l'intervalle allant de 2,5 à 4,5,
- et un détergent choisi parmi le dodécylsulfate de sodium (SDS), le dodécylsulfate de lithium, le dodécylsulfate de potassium, le cholate de sodium, le désoxycholate de sodium, l'octylglucoside, l'oxyde de dodécyl-diméthylamine, le 3-[(3-cholamidopropyl)diméthylammonio]-1-propane-sulfonate (CHAPS), le bromure de dodécyl-triéthylammonium (DTAB), le bromure de cétyl-triméthylammonium (CTAB) et un polyéther p-isooctylphénol polyéthoxylé,
pour traiter un matériau en vue d'annihiler l'infectiosité de prions infectieux.

21. Procédé conforme à la revendication 1, 9, 12 ou 16, dans lequel le polyéther p-isooctylphénol polyéthoxylé est du Triton X-20, du Triton X-100 ou du Triton X-114.

22. Composition conforme à la revendication 18, dans laquelle le polyéther p-isooctylphénol polyéthoxylé est du Triton X-20, du Triton X-100 ou du Triton X-114.

23. Emploi conforme à la revendication 20, dans lequel le polyéther p-isooctylphénol polyéthoxylé est du Triton X-20, du Triton X-100 ou du Triton X-114.
